(19) Europäisches Patentamt / European Patent Office / Office européen des brevets

(11) **EP 4 502 568 A1**

(12) **EUROPEAN PATENT APPLICATION**
published in accordance with Art. 153(4) EPC

(43) Date of publication:
**05.02.2025 Bulletin 2025/06**

(21) Application number: **23779125.6**

(22) Date of filing: **27.02.2023**

(51) International Patent Classification (IPC):
*G01N 1/04* (2006.01)     *B03B 5/00* (2006.01)
*G01N 37/00* (2006.01)

(52) Cooperative Patent Classification (CPC):
**B03B 5/00; G01N 1/04; G01N 37/00**

(86) International application number:
**PCT/JP2023/007099**

(87) International publication number:
**WO 2023/189095 (05.10.2023 Gazette 2023/40)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **28.03.2022 JP 2022051142**

(71) Applicants:
• **NATIONAL UNIVERSITY CORPORATION
  IBARAKI UNIVERSITY**
  **Mito-shi, Ibaraki 310-8512 (JP)**
• **Dinow Inc.**
  **Mito-shi, Ibaraki 310-0021 (JP)**
• **NOK Corporation**
  **Minato-ku**
  **Tokyo 105-8585 (JP)**

(72) Inventors:
• **NAKAMURA, Asako**
  **Mito-shi, Ibaraki 310-8512 (JP)**
• **TAKAHASHI, Kenta**
  **Mito-shi, Ibaraki 310-0021 (JP)**
• **CHIHARA, Yuma**
  **Fujisawa-shi, Kanagawa 251-0042 (JP)**
• **KOMORI, Takayuki**
  **Fujisawa-shi, Kanagawa 251-0042 (JP)**
• **FUJISAWA, Naohiro**
  **Fujisawa-shi, Kanagawa 251-0042 (JP)**

(74) Representative: **Global IP Europe
Patentanwaltskanzlei
Pfarrstraße 14
80538 München (DE)**

(54) **WHITE BLOOD CELL CAPTURING DEVICE**

(57) The present invention addresses the problem of providing a white blood cell capturing device that captures white blood cells with high efficiency while being unsusceptible to capturing other material. The problem is solved by this white blood cell capturing device, which captures white blood cells included in a blood-containing liquid, and includes a sample entrance, a buffer entrance, a white blood cell separation unit, a main part, an auxiliary part, and an exit, wherein: the main part has a flat portion and a large number of protrusions provided thereon, the main part being configured such that a buffer which has entered from the entrance passes above the main part and is discharged from the exit; the protrusions are provided in layered form on the surface of the flat portion, each layer includes a plurality of the protrusions, and the protrusions are configured such that a buffer that has passed through a layer on the entrance side passes through a layer on the exit side adjacent thereto; and the capture part faces the exit side of all or some of the bypass parts in a specific layer, and is disposed as a part of a separate layer adjacent thereto.

EP 4 502 568 A1

**(Cont. next page)**

FIG.1

**Description**

TECHNICAL FIELD

The present invention relates to a white blood cell capturing device.

BACKGROUND ART

DNA is damaged by the influence of radiation exposure or living environment. It has been considered that the damaged DNA is closely related to diseases such as cancers. A method which includes centrifuging blood to isolate white blood cells, staining the isolated white blood cells, and then observing on a glass slide has heretofore been adopted to analyze the damaged **DNA.**

A micro flow path for capturing fine particles such as white blood cells has heretofore been proposed (see Patent Document 1). Patent Document 1 discloses a micro flow path device which has a filter function for only capturing and separating solid particles having at least a predetermined size from a solid-liquid mixture using a micro flow path having concave capturing portions. Patent Document 1 aims at receiving one or more solid particles having at least a predetermined size in one capturing portion to completely capture solid particles having at least the predetermined size before the distal end of a separating part including the plurality of capturing portions disposed therein, and does not consider capturing only one solid particle in a single capturing portion so that the captured solid particle such as a white blood cell may be easily observed. Further, the solid-liquid mixture needs to flow at all times from an inlet of the micro flow path toward its outlet to prevent solid particles once captured in the capturing portions from refloating and flowing out of the capturing portions.

CITATION LIST

PATENT DOCUMENTS

Patent Document 1: JP 2009-109232 A

SUMMARY OF THE INVENTION

PROBLEM TO BE SOLVED BY THE INVENTION

In the conventional method, however, red blood cells, proteins and the like are contained in a liquid from which white blood cells are to be captured, and their coagulates are often captured together with the white blood cells to cause noises when they are observed.

An object of the present invention is to solve the problem as described above. Specifically, an object of the present invention is to provide a white blood cell capturing device which captures white blood cells at a high degree of efficiency and is less likely to capture the other components.

MEANS FOR SOLVING THE PROBLEM

The present invention provides the following (1) and (6).

(1) A white blood cell capturing device configured to capture white blood cells contained in a blood-containing liquid by passing the blood-containing liquid therethrough, the device including:

a sample inlet into which the blood-containing liquid X1 is to be introduced;
a buffer inlet into which a buffer Y1 is to be introduced;
a white blood cell separation unit:

which includes a flat portion and a plurality of columns extending in a perpendicular line direction of the flat portion, and is configured so that the blood-containing liquid X1 and the buffer Y1 flow in a specific same direction Z so as to be adjacent to each other on a surface of the flat portion and between the columns adjacent to each other,
in which, when the surface of the flat portion is seen from a direction parallel to its perpendicular line, the columns form a plurality of lines and are arranged so that each line forms an angle θ with respect to the same direction Z in which the blood-containing liquid X1 and the buffer Y1 flow,
in which in a configuration using Deterministic Lateral Displacement (DLD) where a distance between one column and another column adjacent thereto in a direction perpendicular to the same direction Z in a case where the surface of the flat portion is seen from the direction parallel to its perpendicular line is represented by g, a threshold diameter Dc defined by $Dc = 1.4 \, g \times (1/\tan\theta)^{-0.48}$ is smaller than a diameter of the white blood cells, and
which is configured to separate the white blood cells from the blood-containing liquid X1 to be contained in the buffer Y1 in a process in which the blood-containing liquid X1 and the buffer Y1 flow in the specific same direction **Z,** whereby a buffer Y2 containing the white blood cells and a blood-containing liquid X2 as a remainder after separation of the white blood cells are produced and discharged;

a main part configured to pass the buffer Y2 discharged from the white blood cell separation unit therethrough to capture the white blood cells contained therein;
an auxiliary part configured to pass the blood-containing liquid X2 discharged from the white blood cell separation unit therethrough, the auxiliary part being identical in flow path resistance to the main part; and an outlet configured to discharge liquids after having passed through the main part and the auxiliary part,
wherein:

the main part has a flat portion and a large number of protruding portions provided thereon, and is configured so that the buffer Y2 having entered through an inlet passes on the surface of the flat portion at the main part and through spaces each located between a protruding portion and another protruding portion adjacent thereto and is discharged from its outlet,
the protruding portions are formed in layers on the surface of the flat portion, each layer has a plurality of protruding portions, and the protruding portions are configured so that the buffer Y2 having passed through a layer on an inlet side passes through a layer adjacent thereto on an outlet side,
capturing portions and bypass portions are formed in each layer, each of the capturing portions has a width set to 2 to 7.5 $\mu$m between a protruding portion and another protruding portion adjacent thereto, and each of the bypass portions has a width set to 8 to 20 $\mu$m therebetween,
inlet side portions on an inlet side of two protruding portions constituting one capturing portion are chamfered so that a width therebetween is gradually reduced toward a bottom of the capturing portion, and
capturing portions are located so as to face an outlet side of all or some bypass portions in a specific layer as a part of another layer adjacent thereto.

(2) The white blood cell capturing device according to (1) above, wherein the auxiliary part is identical in configuration to the main part.
(3) The white blood cell capturing device according to (1) or (2) above, wherein a width between the specific layer and the another layer adjacent thereto in the main part is 8 to 30 $\mu$m.
(4) The white blood cell capturing device according to any one of (1) to (3) above, wherein a ratio of a bypass portion width to a capturing portion width in the main part is more than 1 but not more than 3.
(5) The white blood cell capturing device according to any one of (1) to (4) above, wherein in the main part, portions of the protruding portions at their inlet side end faces except the capturing portions extend parallel to a layer direction, and end faces of the protruding portions constituting the bypass portions extend in a direction perpendicular to the layer direction.
(6) The white blood cell capturing device according to any one of (1) to (5) above, wherein the threshold diameter Dc is 5 $\mu$m or more but less than 10 $\mu$m.

EFFECT OF THE INVENTION

The present invention can provide a white blood cell capturing device which captures white blood cells at a high degree of efficiency and is less likely to capture the other components.

BRIEF DESCRIPTION OF THE DRAWINGS

[FIG. 1] FIG. 1 is a schematic plan view showing a white blood cell capturing device of the invention.
[FIG. 2] FIG. 2 is a cross-sectional view (schematic cross-sectional view) taken along line A-A in FIG. 1.
[FIG. 3] FIG. 3 is an enlarged view of a binding portion between flow paths 5 and 6, and an inlet 10in of a white blood cell separation unit 10.
[FIG. 4] FIG. 4 is an enlarged view (schematic view) of a surface in FIG. 1.
[FIG. 5] FIG. 5 is a schematic view of a surface of a main part 50 when seen from a direction parallel to its perpendicular line.
[FIG. 6] FIG. 6 is an enlarged view (schematic view) of B in FIG. 5.
[FIG. 7] FIG. 7 is a cross-sectional view taken along line C-C in FIG. 5.
[FIG. 8] FIG. 8 is a micrograph (combination of a 10x ocular lens and a 10x objective lens) of the white blood cell capturing device of the invention according to EXAMPLES.

DESCRIPTION OF EMBODIMENTS

The white blood cell capturing device of the invention will be described.
The white blood cell capturing device of the invention flows a blood-containing liquid having entered through an inlet toward an outlet by the action of a pump (syringe pump or the like), hydrostatic pressure, electroosmotic flow or the like. Then, during this process, white blood cells are moved from the interior of the blood-containing liquid into a buffer and thereafter captured by capturing portions located between protruding portions adjacent to each other in a main part.
The white blood cell capturing device of the invention can be fabricated by, for example, pouring rubber into a mold having a flow path pattern formed therein to obtain a flow path chip, and then attaching the obtained chip to a glass substrate.

The white blood cell capturing device of the invention will be described with reference to drawings.

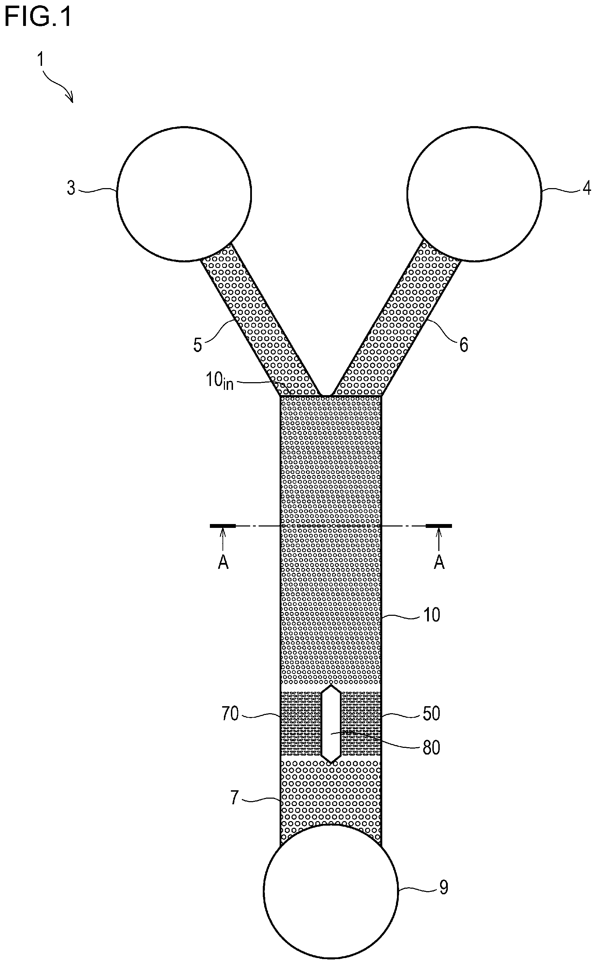

FIG. 1 is a schematic plan view showing a white blood cell capturing device 1 of invention.

The white blood cell capturing device 1 of the invention shown in FIG. 1 is obtained by pouring rubber into a mold having a flow path pattern formed therein to obtain a flow path chip, and then attaching the obtained chip to a glass substrate.

However, a cover covering an upper surface of the white blood cell capturing device 1 of the invention (which will be described later) is not shown in FIG. 1.

The white blood cell capturing device of the invention which will be described below with reference to drawings is a preferred embodiment and is merely illustrative. The white blood cell capturing device of the invention should not be construed as being limited to the embodiment shown in the drawings.

The white blood cell capturing device 1 of the invention has a sample inlet 3, a buffer inlet 4, a white blood cell separation unit 10, a main part 50, an auxiliary part 70, and an outlet 9.

<Sample Inlet>

A blood-containing liquid X1 is introduced into the sample inlet 3.

The sample inlet 3 may be a hole where the introduced blood-containing liquid X1 can temporarily remain.

According to the embodiment shown in FIG. 1, for example, a hole where the blood-containing liquid X1 can temporarily remain is formed by pouring rubber into a mold having a flow path pattern formed therein to obtain a flow path chip, forming a through-hole in the flow path chip, and attaching the flow path chip to a glass substrate, and the thus formed hole can be used as the sample inlet 3.

A flow path 5 connects the sample inlet 3 to an inlet 10in of the white blood cell separation unit 10, and configuration is made so that the blood-containing liquid X1 having been introduced into the sample inlet 3 passes through the flow path 5 to be introduced into the interior of the white blood cell separation unit 10 from the inlet 10in.

The flow path 5 may be formed of, for example, a pillar. The pillar prevents the flow path 5 from getting clogged when a through-hole is formed in a flow path chip obtained by pouring rubber into a mold having a flow path pattern formed therein, and the flow path chip is attached to a glass substrate.

The blood-containing liquid X1 as used herein is not particularly limited as long as it is a liquid containing blood of animals including humans, and may be, for example, a liquid mixture obtained by adding human blood to a phosphate buffer solution (PBS), an anticoagulant, a stain solution, and the like. The blood-containing liquid may be blood itself of animals including humans.

<Buffer Inlet>

A buffer Y1 is introduced into the buffer inlet 4.

The buffer inlet 4 may be a hole where the introduced buffer Y1 can temporarily remain.

According to the embodiment shown in FIG. 1, for example, a hole where the buffer Y1 can temporarily remain is formed by pouring rubber into a mold having a flow path pattern formed therein to obtain a flow path chip, forming a through-hole in the flow path chip, and attaching the flow path chip to a glass substrate, and the thus formed hole can be used as the buffer inlet 4.

A flow path 6 connects the buffer inlet 4 to the inlet 10in of the white blood cell separation unit 10, and configuration is made so that the buffer Y1 having been introduced into the buffer inlet 4 passes through the flow path 6 to be introduced into the interior of the white blood cell separation unit 10 from the inlet 10in.

As with the flow path 5, the flow path 6 may be formed of, for example, a pillar.

The buffer Y1 is not particularly limited as long as it is a reagent for use in DNA damage analysis, and may be, for example, a phosphate buffer solution (PBS), paraformaldehyde (PFA), DAPI or the like.

<White Blood Cell Separation Unit>

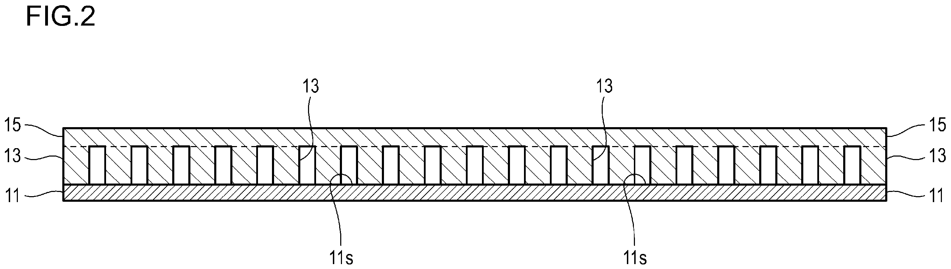

The white blood cell separation unit 10 will be described with reference to FIG. 2, FIG. 3 and FIG. 4 in addition to FIG. 1.

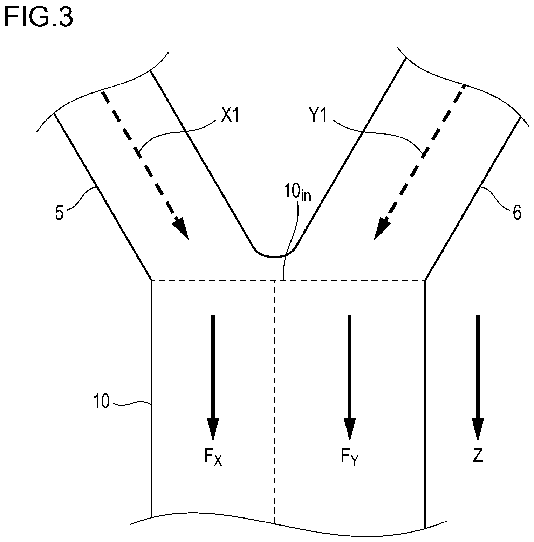

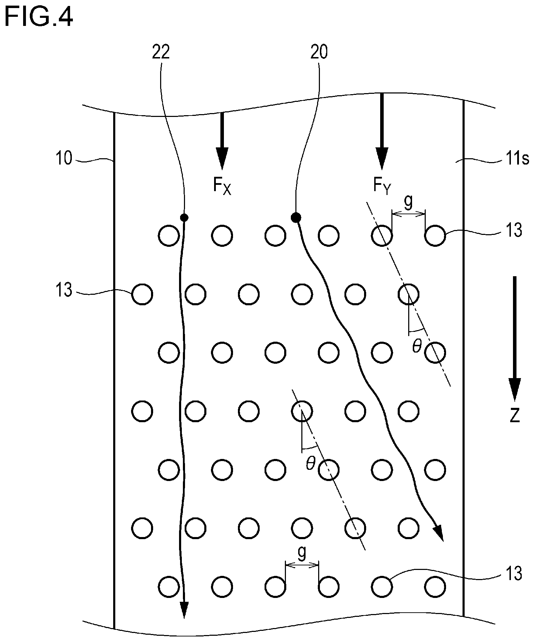

FIG. 2 is a cross-sectional view (schematic cross-sectional view) taken along line A-A in FIG. 1; FIG. 3 is an enlarged view of a binding portion between the flow paths 5 and 6, and the inlet 10in of the white blood cell separation unit 10; and FIG. 4 is an enlarged view (schematic view) of a surface of the white blood cell separation unit 10 in FIG. 1.

As shown in FIG. 2, the white blood cell separation unit 10 has a flat portion 11 and a plurality of columns 13 extending in a perpendicular line direction of the flat portion 11.

As described above, in this example, the white blood cell capturing device is formed by attaching to a glass substrate a flow path chip obtained by pouring rubber into a mold having a flow path pattern formed therein, and the glass substrate constitutes the flat portion 11.

Further, the white blood cell separation unit 10 shown in FIG 2 has a plate-like cover 15 so as to cover upper parts of the columns 13 (the cover 15 is not shown in FIG. 1 and FIG 4). FIG. 2 shows the boundary between the columns 13 and the cover 15 by a dotted line but the columns 13 and the cover 15 may be formed in an integral manner or in a separable manner. A flow path chip in which the columns 13 and the cover 15 are formed integrally with each other can be obtained by changing the shape of a mold for use in obtaining the flow path chip through pouring of rubber to have a flow path pattern in which the columns 13 and the cover 15 are integral with each other.

The blood-containing liquid X1 and the buffer Y1 flow on a surface 11s of the flat portion 11 and between the columns 13 adjacent to each other.

Further, as shown in FIG. 3, the blood-containing liquid X1 which has reached the inlet 10in of the white blood cell separation unit 10 from the flow path 5 and flowed into the interior of the white blood cell separation unit 10 from the inlet 10in, and the buffer Y1 which has reached the inlet 10in of the white blood cell separation unit 10 from the flow path 6 and flowed into the interior of the white blood cell separation unit 10 from the inlet 10in both flow in a specific direction (same direction Z) inside the white blood cell separation unit 10. In other words, in the white blood cell capturing device 10, a direction $F_X$ in which the blood-containing liquid X1 flows and a direction $F_Y$ in which the buffer Y1 flows are approximately parallel to each other and this direction is referred to as the same direction Z.

Further, the blood-containing liquid X1 and the buffer Y1 each form a generally laminar flow. In FIG. 3, the blood-containing liquid X1 and the buffer Y1 flow so as to be adjacent to each other with respect to a dotted line which is present between an arrow indicating the direction $F_X$ and an arrow indicating the direction $F_Y$, and is parallel to an arrow indicating the same direction Z.

This is mainly due to significantly reduced Reynolds numbers of the blood-containing liquid X1 and the buffer Y1.

FIG. 4 is an enlarged view of the surface 11s of the flat portion 11 and corresponds to a drawing when the flat portion 11 is seen from a direction parallel to a perpendicular line of the surface 11s. As shown in FIG. 4, the columns 13 are arranged so as to form a plurality of lines and white blood cells contained in the blood-containing liquid X1 are separated from the other components such as red blood cells and platelets by Deterministic Lateral Displacement (DLD). Blood has white blood cells with a diameter of about 10 to 20 $\mu$m, red blood cells with a diameter of about 7 to 8 $\mu$m, and platelets with a diameter of about 2 to 3 $\mu$m. The white blood cells are the largest in diameter and can be therefore separated by the DLD.

The DLD is disclosed in Naotomo Tottori et al./three others, Development of Deterministic Lateral Displacement Device for Separation of Particles, Proceedings of 2015 Spring Meeting of the Japan Society for Precision Engineering, JSPE, 2015, pp. 743-744; and WO 2016/136273. The DLD is a technique for separating large-sized particles and small-sized particles from a flow of a liquid containing particles dispersed therein making use of a large number of columns regularly arranged in a microfluidic device.

As shown in FIG. 4, the columns 13 form the plurality of lines and are arranged so that each line forms an angle θ with respect to the same direction Z in which the blood-containing liquid X1 and the buffer Y1 flow.

Then, white blood cells 20 move obliquely along the inclined lines of the columns 13. In contrast, small-sized particles 22 which are smaller in diameter than the white blood cells 20 move in a zig-zag manner with respect to the direction of flow ($F_X$ and the same direction Z) while changing the orientation of flow at the columns 13, but generally move in a linear manner along the laminar flow. Therefore, when the blood-containing liquid X1 and the buffer Y1 flow in the specific same direction Z on the flat portion 11 in the white blood cell capturing device 10 so as to be adjacent to each other, the white blood cells 20 in the blood-containing liquid X1 move to the side of the buffer Y1 to enter the interior of the buffer Y1 from the interface between the laminar flow of the blood-containing liquid X1 and that of the buffer Y1.

According to Development of Deterministic Lateral Displacement Device for Separation of Particles and WO 2016/136273, the threshold diameters Dc of the small-sized particles moving along the direction of flow of the blood-containing liquid X1 ($F_X$ and the same direction Z) and the large-sized particles moving obliquely are determined by formula 1 below:

$$\text{Formula 1} \cdots Dc = 1.4 \; g \cdot N^{-0.48}$$

where g is a distance between adjacent columns 13 in a direction perpendicular to the direction of flow ($F_X$ and the same direction Z) in the drawing when the flat portion 11 is seen from a direction parallel to the perpendicular line of the surface 11s of the flat portion 11 as shown in FIG 4, and N is the number of flow streams and is represented by formula below:

$$N = 1 / \varepsilon$$

where $\varepsilon$ is a row shift fraction, i.e., a shift fraction of the columns 13, and $\varepsilon = \tan\theta$.

According to WO 2016/136273, the threshold diameter Dc is determined by the following formula which is equivalent to formula 1.

$$Dc = 1.4 \; g \cdot \varepsilon^{0.48}$$

Particles having diameters smaller than the threshold diameter Dc generally move along the direction of flow, while particles having diameters larger than the threshold diameter Dc move obliquely. As described above, the white blood cells 20 are larger in diameter than the other components. Therefore, the white blood cells 20 can be obliquely moved along the inclined lines of the columns 13 to reach the interior of the buffer Y1 by setting the threshold diameter Dc defined by Dc = 1.4g × (1/tanθ)$^{-0.48}$ to be smaller than the diameter of the white blood cells but larger than that of the other particles (such as platelets) to be separated therefrom.

By setting the threshold diameter Dc to, for example, 5 $\mu$m or more but less than 10 $\mu$m, the threshold diameter Dc becomes smaller than the diameter of the white blood cells (about 10 to 20 $\mu$m) and larger than that of the other particles (such as platelets) to be separated therefrom, and the white blood cells can be separated with a high degree of efficiency.

In FIG. 4, the number and positions of the columns 13 are not necessarily exact. In FIG. 4, the columns 13 have a circular cross-sectional shape but may have any other cross-sectional shapes as long as they exert the separation effect.

The buffer Y1 containing the white blood cells separated as above from the blood-containing liquid X1 is hereinafter referred to as a buffer Y2.

The remainder of the blood-containing liquid X1 from which the white blood cells have been separated is hereinafter referred to as a blood-containing liquid X2.

Then, each of them is discharged from the white blood cell separation unit 10.

The buffer Y2 discharged from the white blood cell separation unit 10 is moved toward the main part to be described later.

The blood-containing liquid X2 discharged from the white blood cell separation unit 10 is moved toward the auxiliary part to be described later.

< Main Part>

The main part 50 serves as a chip for capturing white blood cells contained in the buffer Y2 while passing the buffer Y2 therethrough.

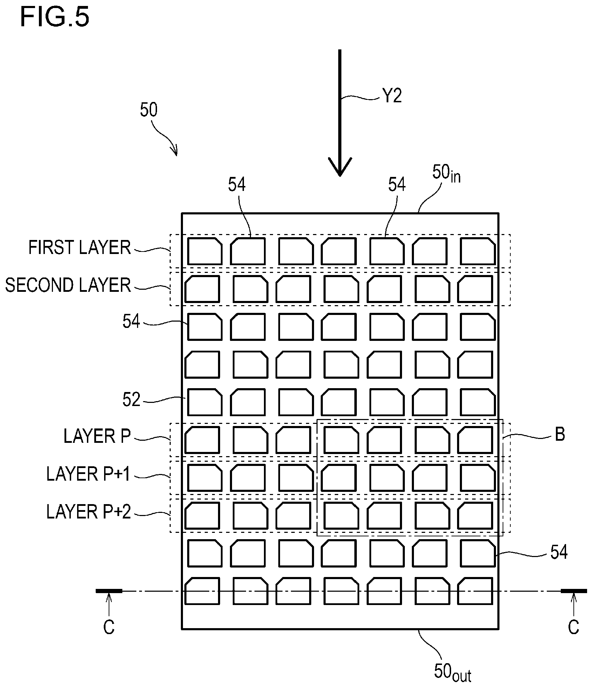

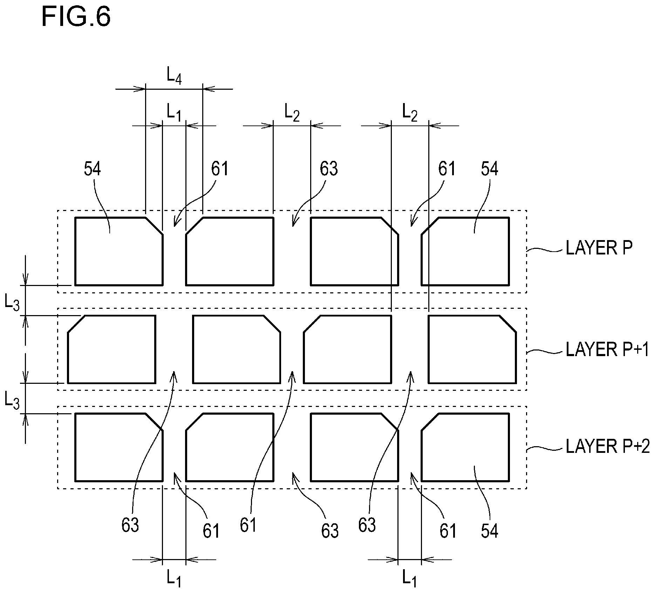

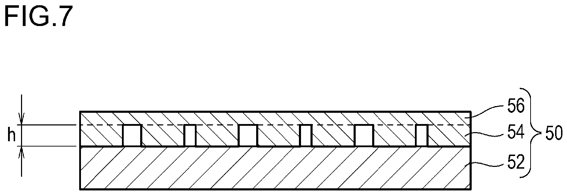

The main part 50 is set with reference to FIG. 5 to FIG. 7. FIG. 5 is a schematic view of a surface of the main part 50 when seen from a direction parallel to its perpendicular line. FIG. 6 is an enlarged view (schematic view) of B in FIG. 5. FIG. 7 is a cross-sectional view taken along line C-C in FIG. 5.

However, a cover covering an upper surface of the main part 50 (which will be described later) is not shown in FIG. 5 and FIG. 6.

The main part 50 illustrated in FIG. 5 has an inlet 50in for introducing the buffer Y2 into its interior and an outlet 50out for discharging the buffer Y2 having passed through the main part 50.

The configuration of the main part 50 is not limited to that illustrated in FIG. 5 to FIG. 7.

As shown in FIG. 5 to FIG. 7, the main part 50 includes a flat portion 52 and a large number of protruding portions 54 provided thereon.

As described above, in this example, the white blood cell capturing device is formed by attaching to a glass substrate a flow path chip obtained by pouring rubber into a mold having a flow path pattern formed therein, and the glass substrate constitutes the flat portion 52.

Further, the main part 50 shown in FIG. 5 to FIG. 7 has a plate-like cover 56 so as to cover upper parts of the protruding portions 54 (the cover 56 is not shown in FIG. 5 and FIG 6). FIG. 7 shows the boundary between the protruding portions 54 and the cover 56 by a dotted line but the protruding portions 54 and the cover 56 may be formed in an integral manner or in a separable manner. A flow path chip in which the protruding portions 54 and the cover 56 are formed integrally with each other can be obtained by changing the shape of a mold for use in obtaining the flow path chip through pouring of rubber to have a flow path pattern in which the protruding portions 54 and the cover 56 are integral with each other.

As shown in FIG. 5, the protruding portions 54 are formed in layers on the flat portion 52.

FIG. 5 shows a layer closest to the inlet 50in as a first layer, and a layer adjacent to the first layer on the outlet side (downstream side) as a second layer. FIG. 5 also shows a layer as a layer P, a layer adjacent to the layer P on the outlet side (downstream side) as a layer P+1, and a layer further adjacent thereto on the outlet side (downstream side) as a layer P+2.

Each layer contains a plurality of protruding portions 54. FIG. 5 shows an example in which each layer contains seven protruding portions 54. However, the number of the protruding portions 54 contained in each layer is not particularly limited. Further, the number of layers is also not particularly limited.

The buffer Y2 having entered the main part 50 from the inlet 50in flows on a surface of the flat portion 52, and first passes through flow paths between the protruding portions 54 in the first layer and then passes through flow paths between the protruding portions 54 in the second layer. The device is configured so that the buffer Y2 flows thereafter in the same manner to pass through flow paths between the protruding portions 54 in the layer P, and then pass through flow paths between the protruding portions 54 in the layer P+1.

As shown in FIG. 6, capturing portions 61 and bypass portions 63 are formed in each layer, each capturing portion 61 having a width (flow path width) $L_1$ set to 2 to 7.5 $\mu$m between a protruding portion 54 and its adjacent protruding portion 54, and each bypass part 23 having a width $L_2$ set to 8 to 20 $\mu$m therebetween.

In the example of FIG. 6, the capturing portions 61 and the bypass portions 63 are alternately formed as flow paths between the plurality of protruding portions 54 in each layer of the layer P, the layer P+1, and the layer P+2. In the main part 50, however, the capturing portions and the bypass portions formed in each layer may not be alternately formed as in FIG. 6. For example, a plurality of capturing portions may be continuously present in each layer.

Further, a capturing portion 61 is located on the outlet side of a bypass portion 63 in a specific layer as a part of another layer adjacent thereto. That is, in the example of FIG. 6, a capturing portion 61 in the layer P+1 is located on the outlet side (downstream side) of a bypass portion 63 in the layer P.

As illustrated in FIG. 6, a bypass portion 63 in the layer P and a capturing portion 61 in the layer P+1 are preferably located side by side in a direction perpendicular to the layer direction. More specifically, the bypass portion 63 in the layer P and the capturing portion 61 in the layer P+1 are preferably located so that, when a straight line in a direction perpendicular to the layer direction is drawn, the straight line passes through the bypass part 63 in the layer P and the capturing portion 61 in the layer P+1 (in other words, the straight line does not come into contact with the protruding portions 54).

In the example shown in FIG. 5 and FIG. 6, white blood cells which are contained in the buffer Y2 having flowed from the inlet side (upstream side) to reach the layer P are in principle not allowed to pass through the capturing portions 61 and at least some of the white blood cells are therefore captured in the capturing portions 61 of the layer P. When the white blood cells are captured, the corresponding capturing portions 61 are closed. On the other hand, even if components other than the white blood cells (red blood cells, platelets, and the like) are contained in the buffer Y2, they are not captured by the capturing portions 61 in the layer P but pass through the capturing portions 61 or the bypass portions 63 to reach the layer P+1. Further, all the components which are contained in the buffer Y2 having reached the layer P can pass through the bypass portions 63. Therefore, white blood cells which were not captured by the capturing portions 61 in the layer P pass through the bypass portions 63 in the layer P to reach the layer P+1, and at least some of them are captured by the capturing portions 61 in the layer P+1. Because the capturing portions 61 in the layer P+1 are located on the outlet side (downstream side) of the bypass portions 63 in the layer P, the white blood cells having passed through the bypass portions 63 in the layer P are easily captured by the capturing portions 61 in the layer P+1.

In the plan views as shown in FIG. 5 and FIG. 6, each protruding portion 54 has a rectangular-based shape in which part of edges in four corners are (for example linearly or curvilinearly) cut off to be chamfered. The chamfered portion which is (for example linearly or curvilinearly) cut off preferably has a smaller area than the area (projected area) of a white blood cell to be captured.

Further, as illustrated in FIG. 6, inlet side portions in two protruding portions 54 constituting a capturing portion 61 are chamfered so that the capturing portion 61 is continuously and gradually narrowed toward its bottom, because in this case, a white blood cell is easily captured by the capturing portion 61 and a white blood cell once captured by the capturing portion 61 is fitted into the chamfered portions in a deformed shape and is therefore less likely to flow out of the capturing portion.

When a part of each edge is linearly cut off to be chamfered, the angle of the line formed by chamfering is preferably 30 to 60° with respect to the direction perpendicular to the layer direction (direction from the inlet toward the outlet). In a case where chamfering is not linear but is, for example, spoon-shaped chamfering or round chamfering, the tangent line preferably forms an average angle of 30 to 60°. When this angle is smaller than 30°, white blood cells tend to flow into the bypass portions 63 at higher speeds to lower the capturing efficiency. When this angle is larger than 60°, the possibility that a plurality of white blood cells are captured in a single capturing portion 61 tends to be increased.

If a capturing portion 61 is gradually narrowed toward its bottom, both inlet side portions of two protruding portions constituting the capturing portion 61 may be chamfered or only one inlet side portion may be chamfered. When both the inlet side portions are chamfered, the chamfering angle may be the same or different.

In a case where the protruding portions 54 have a rectangular-based shape in which part of edges in four corners are (for example linearly or curvilinearly) cut off to be chamfered, other white blood cells that reached the capturing portions 61 already having white blood cells captured therein move in the layer direction along end faces of the protruding portions 54 and move from the bypass portions 63 to the adjacent layer on the downstream side, where the white blood cells are easily captured by the capturing portions 61 in the downstream side layer. Consequently, the inventors have found that the white blood cell capturing efficiency is increased.

As shown in FIG. 6, in the main part 50, portions of the protruding portions 54 at their inlet side end faces except the capturing portions 61 extend parallel to the layer direction, and end faces of the protruding portions 54 constituting the bypass portions 63 extend in a direction perpendicular to the layer direction.

In particular, when as in the case shown in FIG. 6, inlet side portions of two protruding portions 54 constituting each capturing portion 61 are chamfered (preferably linearly chamfered) so that the capturing portion 61 is continuously and gradually narrowed toward its bottom, portions of the protruding portions 54 at their inlet side end faces except the capturing portions 61 extend parallel to the layer direction, and the bypass portions 63 extend in the direction perpendicular to the layer direction, this effect is prominent and the white blood cell capturing efficiency is further increased, and this case is therefore preferable.

In a case where the protruding portions 54 do not have a rectangular-based shape (in the case of a circular shape or an elliptical shape, for example), their outer shape contains R and white blood cells may therefore move along the R instead of moving to the capturing portions 61 in the adjacent layer on the downstream side.

Each of the capturing portions 61 has a width $L_1$ of 2 to 7.5 $\mu$m, preferably 3 to 6 $\mu$m, and more preferably 4 to 5 $\mu$m.

Each of the bypass portions 63 has a width $L_2$ of 8 to 20 $\mu$m, preferably 8.5 to 15 $\mu$m, and more preferably 9 to 10 $\mu$m.

Each of the width $L_1$ and the width $L_2$ means the shortest distance between one protruding portion 54 and its adjacent protruding portion 54 in each layer.

The ratio ($L_2/L_1$) of the width $L_2$ of the bypass portions 63 to the width $L_1$ of the capturing portions 61 is preferably more than 1 but not more than 3, and more preferably 1.5 to 2.5, because in this case, the flow toward the bypass portions 63 is adequately suppressed, thus facilitating capturing of white blood cells in the capturing portions.

The width $L_3$ between the layer P and the layer P+1 is preferably 8 to 30 $\mu$m, and more preferably 9 to 10 $\mu$m.

The width $L_3$ means the shortest distance between the layer P and the layer P+1.

The maximum width $L_4$ of each capturing portion 61 at the chamfered portions on the inlet side is preferably 10 to 35 $\mu$m and more preferably 15 to 25 $\mu$m.

Each of the protruding portions 54 shown in FIG. 7 preferably has a height h of 8 to 30 $\mu$m and more preferably 9 to 15 $\mu$m.

<Auxiliary Part>

The auxiliary part 70 serves to pass the blood-containing liquid X2 discharged from the white blood cell separation unit 10 therethrough. The auxiliary part 70 is configured to have the same flow path resistance as the above-mentioned main part 50.

The same flow path resistance as used herein means that the difference between the pressure loss of the main part from its inlet to its outlet and that of the auxiliary part from its inlet to its outlet is within 20% (preferably within 10%) with respect to the higher pressure loss.

The auxiliary part 70 preferably has the same configuration as the above-mentioned main part 50, When the auxiliary part 70 is configured in the same manner as the above-mentioned main part 50, the flow path resistances to the buffer Y2 and the blood-containing liquid X2 are made identical, and as a result, the flow rates of the blood-containing liquid X1 and the buffer Y1 flowing in the white blood cell separation unit 10 can be controlled to be the same to increase the white blood cell separation efficiency in the white blood cell separation unit 10.

The white blood cell capturing device 1 of the invention preferably has a partition wall 80 between the main part 50 and the auxiliary part 70 for separating them from each other.

The shape or the like of the partition wall 80 is not particularly limited as long as it has the function of separating the main part 50 and the auxiliary part 70 from each other.

<Outlet>

Liquids after having passed through the main part 50 and the auxiliary part 70 pass through a flow path 7 to be discharged from the outlet 9.

The outlet 9 may be a hole through which the liquids after having passed through the main part 50 and the auxiliary part 70 can be discharged.

According to the embodiment shown in FIG. 1, for example, a hole through which the liquids after having passed through the main part 50 and the auxiliary part 70 can be discharged is formed by pouring rubber into a mold having a flow path pattern formed therein to obtain a flow path chip, forming a through-hole in the flow path chip, and attaching the flow path chip to a glass substrate, and the thus formed hole can be used as the outlet 9.

The flow path 7 connects the outlet 50out of the main part 50 and an outlet of the auxiliary part 70 to the outlet 9, and configuration is made so that the liquids discharged from the main part 50 and the auxiliary part 70 pass through the flow path 7 to reach the outlet 9.

The flow path 7 need only be formed of a member having the same function as the above-mentioned flow paths 5 and 6. The flow path 7 may be formed of, for example, a pillar.

As described above, the white blood cell capturing device 1 of the invention may be the one obtained by pouring rubber into a mold having a flow path pattern formed therein to obtain a flow path chip, and then attaching the obtained chip to a glass substrate. The size and the material of the flow path chip are not particularly limited. The flow path chip may be made of, for example, resins such as silicone rubber, acrylic resin, polycarbonate, cyclic olefin polymer, cyclic olefin copolymer, polystyrene, polyethylene, and polyethylene terephthalate. The substrate to which rubber is attached is preferably made of glass but may be made of a material other than glass.

The white blood cell capturing device of the invention as described above only separates white blood cells from a blood-containing liquid toward a buffer and then captures the white blood cells. In other words, when the white blood cells are captured, red blood cells, proteins and the like are not contained in the buffer containing them. Therefore, capturing of these coagulates and the like together with the white blood cells can be avoided. Accordingly, it is not necessary to remove unnecessary blood or clean the interiors of the flow paths.

EXAMPLES

<Fabrication of Main Part>

First, silicone rubber (SILPOT184 manufactured by Dow Corning Corp.) was poured into a mold having a flow path pattern formed therein.

Next, the silicone rubber was vulcanized under conditions of 120°C and 30 minutes.

Next, the silicon rubber was peeled off from the silicon wafer to form a chip having flow paths formed therein.

Next, a punch was used to form holes serving as the sample inlet 3, the buffer inlet 4, and the outlet 9, respectively.

<Joining>

The chip having the flow paths formed therein which was perforated as described above and a glass substrate were both irradiated with vacuum ultraviolet light (L12530-01 manufactured by Hamamatsu Photonics K.K.) for 15 seconds. Then, the irradiated surfaces were bonded together to obtain the white blood cell capturing device 1 of the invention shown in FIG. 1.

<Experiments>

Peripheral blood obtained from an adult male wad diluted 10 times with PBS to obtain the blood-containing liquid X1. PBS was used as the buffer Y1.

Next, the blood-containing liquid X1 was dropped into the sample inlet 3 and the buffer Y1 was dropped into the buffer inlet 4.

Next, the liquids were made to flow by the negative pressure of a syringe pump from the outlet 9.

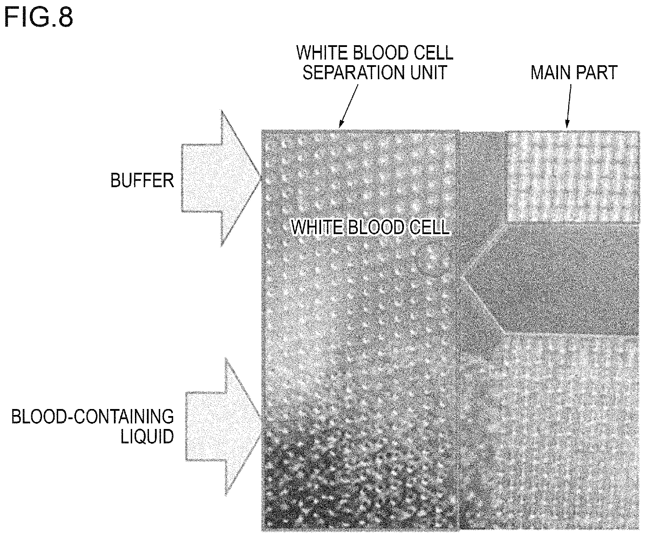

Then, the state of flow of the liquids was checked by a microscope. A micrograph (combination of a 10x ocular lens and a 10x objective lens) is shown in FIG. 8. As shown in FIG. 8, it was revealed that the blood-containing liquid X1 and the buffer Y1 each flowed while forming a laminar flow. It was also revealed that the blood-containing liquid X2 did not flow into the main part 50. It was further revealed that white blood cells were separated from the blood-containing liquid X1 and transferred to the buffer Y1.

The liquids were continuously made to flow for further 30 minutes but clogging of the flow path in the main part 50 was not seen.

It was also revealed that the white blood cells were captured in the main part 50.

This application claims priority based on Japanese Patent Application No. 2022-51142 filed on March 28, 2022, the entire disclosure of which is incorporated herein by reference.

REFERENCE SIGNS LIST

| 1 | white blood cell capturing device of the invention |
| 3 | sample inlet |
| 4 | buffer outlet |
| 5,6,7 | flow paths |
| 9 | outlet |
| 10 | white blood cell separation unit |
| 10in | inlet of the white blood cell separation unit |
| 11 | flat portion |
| 11s | surface of the flat portion |
| 13 | column |
| 15 | cover |
| 20 | white blood cell |
| 22 | small-sized particle |
| 50 | main part |
| 50in | inlet of the main part |
| 50out | outlet of the main part |
| 52 | flat portion |
| 54 | protruding portion |
| 56 | cover |
| 61 | capturing portion |
| 63 | bypass portion |
| 70 | auxiliary part |
| 80 | partition wall |

**Claims**

1. A white blood cell capturing device configured to capture white blood cells contained in a blood-containing liquid by passing the blood-containing liquid therethrough, the device comprising:

   a sample inlet into which the blood-containing liquid X1 is to be introduced;
   a buffer inlet into which a buffer Y1 is to be introduced;
   a white blood cell separation unit:

   which includes a flat portion and a plurality of columns extending in a perpendicular line direction of the flat portion, and is configured so that the blood-containing liquid X1 and the buffer Y1 flow in a specific same direction Z so as to be adjacent to each other on a surface of the flat portion and between the columns adjacent to each other,
   in which, when the surface of the flat portion is seen from a direction parallel to its perpendicular line, the columns form a plurality of lines and are arranged so that each line forms an angle $\theta$ with respect to the same direction Z in which the blood-containing liquid X1 and the buffer Y1 flow,
   in which in a configuration using Deterministic Lateral Displacement (DLD) where a distance between one column and another column adjacent thereto in a direction perpendicular to the same direction Z in a case where the surface of the flat portion is seen from the direction parallel to its perpendicular line is represented by g, a threshold diameter Dc defined by $Dc = 1.4\,g \times (1/\tan\theta)^{-0.48}$ is smaller than a diameter of the white blood cells, and
   which is configured to separate the white blood cells from the blood-containing liquid X1 to be contained in the buffer Y1 in a process in which the blood-containing liquid X1 and the buffer Y1 flow in the specific same direction Z, whereby a buffer Y2 containing the white blood cells and a blood-containing liquid X2 as a remainder after separation of the white blood cells are produced and discharged;

a main part configured to pass the buffer Y2 discharged from the white blood cell separation unit therethrough to capture the white blood cells contained therein;

an auxiliary part configured to pass the blood-containing liquid X2 discharged from the white blood cell separation unit therethrough, the auxiliary part being identical in flow path resistance to the main part; and

an outlet configured to discharge liquids after having passed through the main part and the auxiliary part,

wherein:

the main part has a flat portion and a large number of protruding portions provided thereon, and is configured so that the buffer Y2 having entered through an inlet passes on the surface of the flat portion at the main part and through spaces each located between a protruding portion and another protruding portion adjacent thereto and is discharged from its outlet,

the protruding portions are formed in layers on the surface of the flat portion, each layer has a plurality of protruding portions, and the protruding portions are configured so that the buffer Y2 having passed through a layer on an inlet side passes through a layer adjacent thereto on an outlet side,

capturing portions and bypass portions are formed in each layer, each of the capturing portions has a width set to 2 to 7.5 $\mu$m between a protruding portion and another protruding portion adjacent thereto, and each of the bypass portions has a width set to 8 to 20 $\mu$m therebetween,

inlet side portions on an inlet side of two protruding portions constituting one capturing portion are chamfered so that a width therebetween is gradually reduced toward a bottom of the capturing portion, and

capturing portions are located so as to face an outlet side of all or some bypass portions in a specific layer as a part of another layer adjacent thereto.

2. The white blood cell capturing device according to claim 1, wherein the auxiliary part is identical in configuration to the main part.

3. The white blood cell capturing device according to claim 1 or 2, wherein a width between the specific layer and the another layer adjacent thereto in the main part is 8 to 30 $\mu$m.

4. The white blood cell capturing device according to any one of claims 1 to 3, wherein a ratio of a bypass portion width to a capturing portion width in the main part is more than 1 but not more than 3.

5. The white blood cell capturing device according to any one of claims 1 to 4, wherein in the main part, portions of the protruding portions at their inlet side end faces except the capturing portions extend parallel to a layer direction, and end faces of the protruding portions constituting the bypass portions extend in a direction perpendicular to the layer direction.

6. The white blood cell capturing device according to any one of claims 1 to 5, wherein the threshold diameter Dc is 5 $\mu$m or more but less than 10 $\mu$m.

FIG.1

FIG.2

FIG.3

## FIG.4

FIG.5

FIRST LAYER

SECOND LAYER

54

52

LAYER P

LAYER P+1

LAYER P+2

Y2

50

54

54

50in

54

B

C

C

54

50out

# FIG.6

FIG.7

## FIG.8

WHITE BLOOD CELL SEPARATION UNIT

MAIN PART

BUFFER

WHITE BLOOD CELL

BLOOD-CONTAINING LIQUID

## INTERNATIONAL SEARCH REPORT

International application No.

**PCT/JP2023/007099**

### A. CLASSIFICATION OF SUBJECT MATTER

*G01N 1/04*(2006.01)i; *B03B 5/00*(2006.01)i; *G01N 37/00*(2006.01)i
FI: G01N1/04 H; B03B5/00 Z; G01N37/00 101; G01N1/04 G; G01N1/04 M

According to International Patent Classification (IPC) or to both national classification and IPC

### B. FIELDS SEARCHED

Minimum documentation searched (classification system followed by classification symbols)

G01N1/00-1/44; B03B5/00; B01L3/00; G01N33/48; G01N37/00; C12M1/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| A | JP 2012-88055 A (TOKYO UNIV OF AGRICULTURE & TECHNOLOGY) 10 May 2012 (2012-05-10)<br>entire text, all drawings | 1-6 |
| A | JP 2017-921 A (UNIV TOKYO) 05 January 2017 (2017-01-05)<br>entire text, all drawings | 1-6 |
| A | JP 2005-140790 A (STEAG MICROPARTS GMBH) 02 June 2005 (2005-06-02)<br>entire text, all drawings | 1-6 |
| A | JP 2003-102710 A (OTSUKA, Hiroshi) 08 April 2003 (2003-04-08)<br>entire text, all drawings | 1-6 |
| A | JP 2008-538282 A (CELLPOINT DIAGNOSTICS INC) 23 October 2008 (2008-10-23)<br>entire text, all drawings | 1-6 |
| A | US 2009/0194420 A1 (LAWRENCE LIVERMORE NATIONAL SECURITY, LLC.) 06 August 2009 (2009-08-06)<br>entire text, all drawings | 1-6 |

☑ Further documents are listed in the continuation of Box C.     ☑ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **18 April 2023** | **16 May 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**

| | International application No. |
|---|---|
| | **PCT/JP2023/007099** |

**C.     DOCUMENTS CONSIDERED TO BE RELEVANT**

| Category* | Citation of document, with indication, where appropriate, of the relevant passages | Relevant to claim No. |
|---|---|---|
| P, A | WO 2022/070841 A1 (NOK CORPORATION) 07 April 2022 (2022-04-07)<br>entire text, all drawings | 1-6 |

Form PCT/ISA/210 (second sheet) (January 2015)

INTERNATIONAL SEARCH REPORT
Information on patent family members

International application No.

**PCT/JP2023/007099**

| Patent document cited in search report | | | Publication date (day/month/year) | Patent family member(s) | | | Publication date (day/month/year) |
|---|---|---|---|---|---|---|---|
| JP | 2012-88055 | A | 10 May 2012 | (Family: none) | | | |
| JP | 2017-921 | A | 05 January 2017 | (Family: none) | | | |
| JP | 2005-140790 | A | 02 June 2005 | US | 2005/0106756 | A1 | |
| | | | | EP | 1531003 | A1 | |
| | | | | DE | 10352535 | A1 | |
| | | | | CN | 1628907 | A | |
| JP | 2003-102710 | A | 08 April 2003 | (Family: none) | | | |
| JP | 2008-538282 | A | 23 October 2008 | WO | 2006/108087 | A2 | |
| | | | | US | 2007/0026415 | A1 | |
| | | | | US | 2007/0026413 | A1 | |
| | | | | US | 2007/0026418 | A1 | |
| | | | | US | 2007/0026414 | A1 | |
| | | | | US | 2007/0026417 | A1 | |
| | | | | GB | 2429774 | A | |
| | | | | GB | 2427468 | A | |
| | | | | GB | 2472927 | A | |
| | | | | EP | 2492339 | A1 | |
| | | | | EP | 2594631 | A1 | |
| | | | | EP | 2664666 | A2 | |
| | | | | CN | 101918527 | A | |
| US | 2009/0194420 | A1 | 06 August 2009 | (Family: none) | | | |
| WO | 2022/070841 | A1 | 07 April 2022 | (Family: none) | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2009109232 A
- WO 2016136273 A

- JP 2022051142 A

**Non-patent literature cited in the description**

- **NAOTOMO TOTTORI et al.** Development of Deterministic Lateral Displacement Device for Separation of Particles. *Proceedings of 2015 Spring Meeting of the Japan Society for Precision Engineering, JSPE*, 2015, 743-744